# EUROPEAN PATENT APPLICATION

(11) **EP 2 777 501 A1**
(43) Date of publication of application: **17.09.2014**
(21) Application number: 14157016.8
(22) Date of filing: 27.02.2014
(51) Int. Cl.: A61B 6/00, A61B 8/00

(54) **Portable display unit for medical image**

(30) Priority: 14.03.2013 JP 2013052009
(71) Applicant: Fujifilm Corporation, Minato-ku Tokyo (JP)
(72) Inventor: Kudo, Yuya, Tokyo, 106-8620 (JP); Ueda, Satoshi, Tokyo, 106-8620 (JP); Okusu, Kiyohisa, Tokyo, 106-8620 (JP); Matsumasa, Hironori, Tokyo, 106-8620 (JP); Ohta, Yasunori, Kanagawa (JP)
(74) Representative: Klunker . Schmitt-Nilson . Hirsch

(57) **Abstract**

In an image viewing system, a medical image (50) is displayed on a display panel (18) having a predetermined aspect ratio. One of a horizontal orientation and a vertical orientation in which the display panel is positioned is detected. A longitudinal direction of the display panel is vertical in case of the vertical orientation, and is horizontal in case of the horizontal orientation. The medical image is processed in rotation processing relative to the display panel by maintaining a size of the medical image in compliance with one of the horizontal and vertical orientations of the display panel, to orient the medical image in a proper orientation relative to a viewing direction to the display panel. Assuming that a blank area (52) is created outside a display area of the medical image on the display panel after the rotation processing, relevant information (54, 56) relevant to the medical image is displayed in the blank area.

## Description

### BACKGROUND OF THE INVENTION

### 1. Field of the Invention

The present invention relates to a portable display unit for a medical image. More particularly, the present invention relates to a portable display unit in which a medical image is rotated according to rotational shift of a display area and also the display area can be fully utilized.

### 2. Description Related to the Prior Art

A display apparatus for a diagnostic image is widely used. The display apparatus has a display area of a display panel of which a length is different from a width. The display area displays a medical image in a vertical direction in which the display area is vertically long and an horizontal direction in which the display area is horizontally long.

U.S. Pat. No. 2011/301,463 (corresponding to JP-A 2011-254962) discloses the display apparatus including an ultrasonic transducer, an image output device, a display unit and a controller. The ultrasonic transducer scans a wall of a body cavity with an ultrasonic wave. The image output device receives an ultrasonic image from the ultrasonic transducer, and creates the medical image by combining various data with the ultrasonic image. The display unit has the display area for displaying the medical image in a rotatable manner between horizontal and vertical orientations. The controller changes at least one of a scan condition and display condition by detecting a rotational shift to the vertical or horizontal orientations.

Also, U.S. Pat. No. 2011/301,463 discloses an example of display control of the controller. The medical image is rotated in compliance with rotational shift of the display area so as to prevent the medical image from changing in the direction even upon rotational shift of the display area. The medical image is enlarged or reduced according to one of the horizontal and vertical orientations of the display area so as to maximize the medical image within the display area. Also, the document discloses an example in which the display unit is portable.

However, there occurs a problem in that an apparent size of a lesion or the like is changed assuming that a size of the medical image is changed according to an orientation of the display area in the display apparatus of U.S. Pat. No. 2011/301,463. A condition of the lesion cannot be diagnosed correctly.

In general, an aspect ratio of the display area is not 1. The aspect ratio of the display area is changed upon rotational shift of the display area. In case of rotating the medical image according to the rotational shift of the display area without changing the size, it is impossible to display the medical image in the entirety of the display area after the rotational shift. A blank area is created outside the medical image in the display area, which cannot be fully utilized.

### SUMMARY OF THE INVENTION

In view of the foregoing problems, an object of the present invention is to provide a portable display unit in which a medical image is rotated according to rotational shift of a display area and also the display area can be fully utilized.

In order to achieve the above and other objects and advantages of this invention, a portable display unit includes a display panel, having a predetermined aspect ratio, for displaying a medical image. An orientation sensor detects a selected one of a horizontal orientation and a vertical orientation in which the display panel is positioned, wherein a longitudinal direction of the display panel is vertical in case of the vertical orientation, and is horizontal in case of the horizontal orientation. A display control device processes the medical image in rotation processing relative to the display panel by maintaining a size and an object orientation of the medical image assuming that the display panel is rotationally shifted from one of the horizontal and vertical orientations to a remaining one thereof, wherein assuming that a blank area is created outside a display area of the medical image on the display panel after the rotation processing, the display control device displays relevant information relevant to the medical image in the blank area.

Preferably, assuming that an end portion of the medical image protrudes from the display panel after the rotation processing, the display control device causes the display panel to display the medical image after removing the end portion.

Preferably, the display control device resizes the medical image to be displayed on the display panel.

Preferably, the display control device processes the medical image being resized in the rotation processing.

Preferably, the display control device resizes the medical image to display the medical image entirely in the vertical orientation. Assuming that the display panel is in the vertical orientation, the medical image after being resized is displayed. Assuming that the display panel is in the horizontal orientation, the display control device creates a partial image by removing at least one of upper and lower end portions of the medical image after being resized, and causes the display panel to display the partial image after the rotation processing.

Preferably, the relevant information is auxiliary information attached to the medical image being displayed.

Preferably, the relevant information is a localizer, displayed in case the display area of the medical image is partially displayed on the display panel, for indicating a position and size of the display area.

Preferably, the relevant information is a thumbnail image obtained by reducing a size of one or more other medical images related to the medical image being displayed.

Preferably, the relevant information is a link tag for access to a link destination for storing information related to the medical image being displayed.

Preferably, a region of interest is determined in the medical image, and the display control device performs control for the medical image to dispose the region of interest at a center of the display area of the medical image.

Preferably, furthermore, an input interface is externally operable, for determining the region of interest.

Preferably, furthermore, an image analysis device for analyzing the medical image, wherein the region of interest is determined according to analysis in the image analysis device.

Preferably, furthermore, an input interface selectively sets first and second control modes for operating the display control device. In the first control mode, the size of the medical image is fixed in the rotation processing, and in the second control mode, the size of the medical image is adjustable in the rotation processing.

Preferably, in the second control mode, the display control device enlarges or reduces the medical image being displayed according to the aspect ratio of the display panel after the rotation processing, and displays the medical image in a maximized size in the display panel after the rotation processing.

Preferably, furthermore, there is a communication interface for connection to a server apparatus by network connection and receiving transmission of the medical image from the server apparatus.

Preferably, the orientation sensor is a gyro sensor or magnetic sensor.

Preferably, a display method includes a step of displaying a medical image on a display panel having a predetermined aspect ratio. A selected one of a horizontal orientation and a vertical orientation in which the display panel is positioned is detected, wherein a longitudinal direction of the display panel is vertical in case of the vertical orientation, and is horizontal in case of the horizontal orientation. The medical image is processed in rotation processing relative to the display panel by maintaining a size of the medical image in compliance with one of the horizontal and vertical orientations of the display panel, to orient the medical image in a proper orientation relative to a viewing direction to the display panel. Assuming that a blank area is created outside a display area of the medical image on the display panel after the rotation processing, relevant information relevant to the medical image is displayed in the blank area.

Preferably, a computer-executable program for display includes a displaying program code for displaying a medical image on a display panel having a predetermined aspect ratio. A detecting program code is for detecting a selected one of a horizontal orientation and a vertical orientation in which the display panel is positioned, wherein a longitudinal direction of the display panel is vertical in case of the vertical orientation, and is horizontal in case of the horizontal orientation. A processing program code is for processing the medical image in rotation processing relative to the display panel by maintaining a size of the medical image in compliance with one of the horizontal and vertical orientations of the display panel, to orient the medical image in a proper orientation relative to a viewing direction to the display panel. An information displaying program code is for, assuming that a blank area is created outside a display area of the medical image on the display panel after the rotation processing, displaying relevant information relevant to the medical image in the blank area.

Consequently, a display area of the portable display unit can be fully utilized, because relevant information can be displayed in a blank area created after rotation processing.

### BRIEF DESCRIPTION OF THE DRAWINGS

The above objects and advantages of the present invention will become more apparent from the following detailed description when read in connection with the accompanying drawings, in which:
Fig. 1 is a block diagram schematically illustrating a image viewing system or remote diagnostic system (medical support system);
Fig. 2 is a block diagram schematically illustrating a portable display unit;
Fig. 3 is a block diagram schematically illustrating a CPU;
Fig. 4 is a flow chart illustrating a display control of a medical image;
Fig. 5 is a flow chart illustrating a display control after rotational shift of the portable display unit;
Figs. 6A-6E are explanatory views in plans illustrating relationships between the orientation of the portable display unit and a display condition;
Fig. 7 is a flow chart illustrating another preferred display control of a medical image;
Figs. 8A-8D are explanatory views in plans illustrating relationships between the orientation of the portable display unit and a display condition.

### DETAILED DESCRIPTION OF THE PREFERRED EMBODIMENT(S) OF THE PRESENT INVENTION

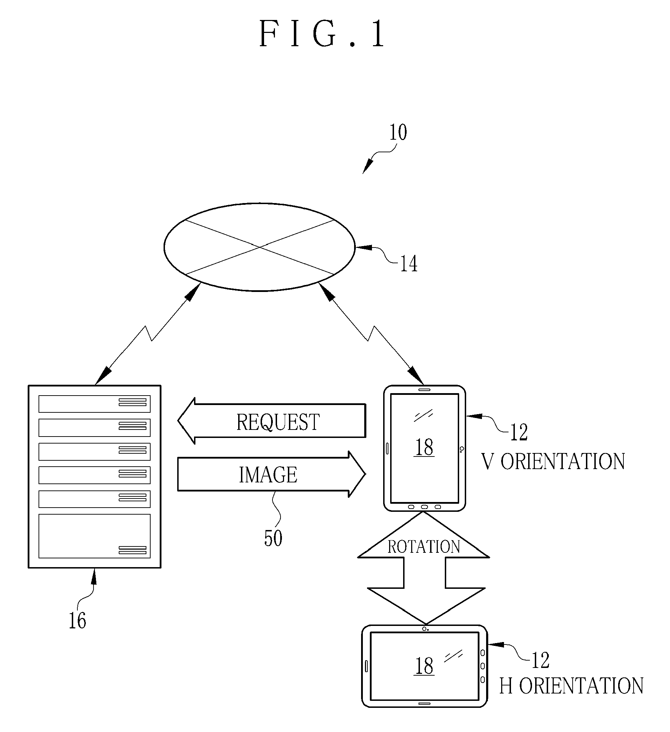

In Fig. 1, an image viewing system 10 or remote diagnostic system (medical support system) includes a portable display unit 12 as a mobile terminal apparatus. There is a server apparatus 16 (for images) or data storage apparatus to which the portable display unit 12 is connected by a communication network 14, such as the Internet, local area network (LAN) and the like. A great number of medical images 50 (diagnostic images) are stored in the server apparatus 16, have been obtained in the course of examination in a hospital or other medical facilities. The server apparatus 16 distributes the medical images 50 to the portable display unit 12 upon receiving a request for the distribution.

The portable display unit 12 transmits information of a request of the medical image 50 to the server apparatus 16. A display panel 18 or display device in the portable display unit 12 displays the medical image 50 transmitted from the server apparatus 16 in response to the request. The display panel 18 is in a quadrilateral shape of an aspect ratio of which a length is different from a width. The portable display unit 12 is used in a vertical orientation in which the display panel 18 is vertically long and in a horizontal orientation in which the display panel 18 is horizontally long. The display panel 18 displays the medical image 50 in each of the horizontal and vertical orientations.

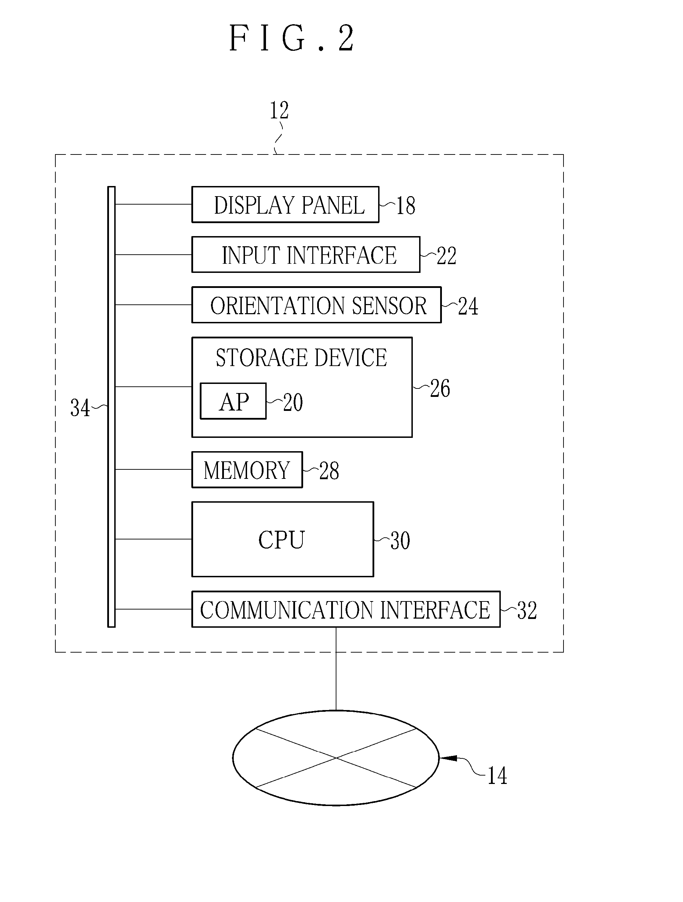

In Fig. 2, various computer-executable programs are installed in a portable computer to constitute the portable display unit 12. Examples of the portable computer include a tablet type, notebook type and the like. Examples of the programs include an application program 20 (AP) for functioning as a display panel, a control program such as an operating system (OS), and the like.

In addition to the display panel 18, the portable display unit 12 includes an input interface 22 or input panel (input interface means), an orientation sensor 24 or detecting sensor, a storage device 26, a memory 28, a CPU 30 (central processing unit) and a communication interface 32. There is a data bus 34 for connecting those elements. The input interface 22 is constituted by various operating buttons and a sensor of a touch panel structure combined with the display panel 18, and detects input signals input by a user or operator to the portable display unit 12. Examples of the orientation sensor 24 are a gyro sensor and a magnetic sensor for detecting the geomagnetic field. The orientation sensor 24 detects one of the horizontal and vertical orientations of the portable display unit 12.

The storage device 26 is such a storage medium as solid-state drive (SSD) or hard disk, and stores the control program. The memory 28 is a working memory with which the CPU 30 performs tasks. The CPU 30 controls various elements in the computer by performing the control according to the control program after loading the memory 28 with the control program. The communication interface 32 is constituted by a network interface for communication control with the communication network 14. The portable display unit 12 communicates with the server apparatus 16 by use of the communication interface 32.

The application program 20 stored in the storage device 26 is software for the display control of diagnostic images. The application program 20 causes the computer to run with a function for transmitting a request of the medical image 50 to the server apparatus 16, and a function for causing the display panel 18 to display the medical image 50 transmitted from the server apparatus 16. The CPU 30 loads the memory 28 with the application program 20 and controls the various elements of the computer by running the software.

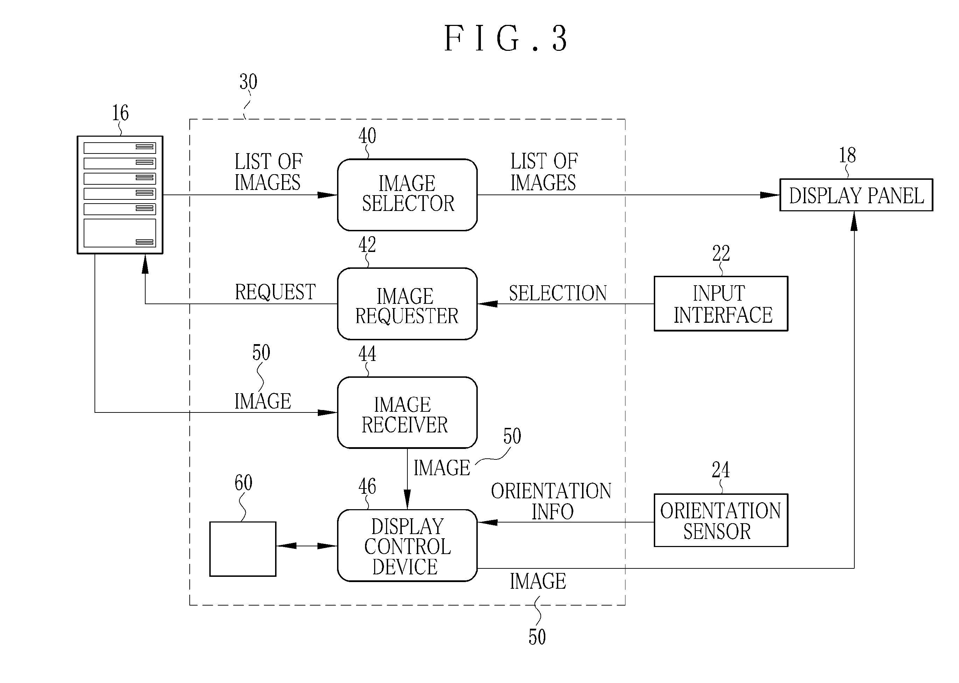

In Fig. 3, the application program 20 for display control is run in the CPU 30 to start various functions in the CPU 30 with the memory 28. In short, the CPU 30 includes an image selector 40, an image requester 42, an image receiver 44 and a display control device 46 (display control means).

The image selector 40 operates in response to startup of the application program 20. The image selector 40 accesses the server apparatus 16, retrieves a list of the medical images 50 stored in the server apparatus 16, and causes the display panel 18 to display the list. A user or operator manipulates the input interface 22 and selects one of the medical images 50 as required for transmission by viewing the list on the display panel 18.

In case the medical image 50 specified for the distribution is selected, the image requester 42 transmits information of a request of distributing the image to the server apparatus 16. The server apparatus 16 upon receiving the request transmits the medical image 50 to the portable display unit 12. The medical image 50 from the server apparatus 16 is received by the image receiver 44.

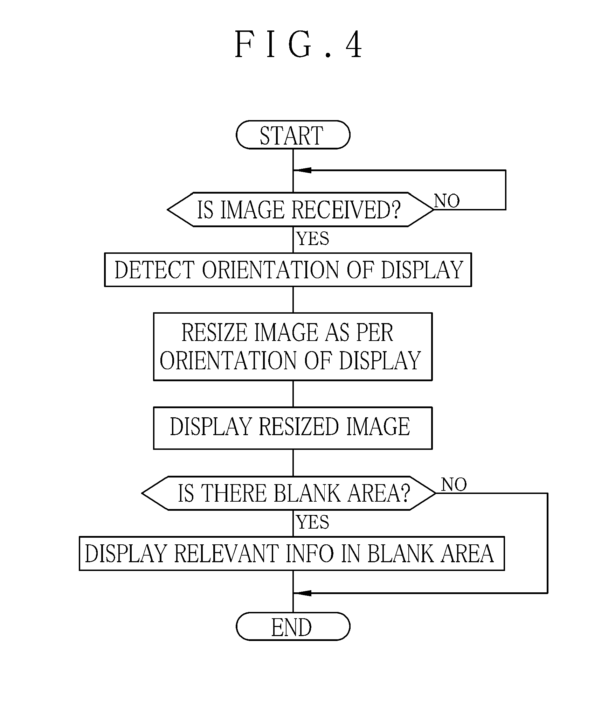

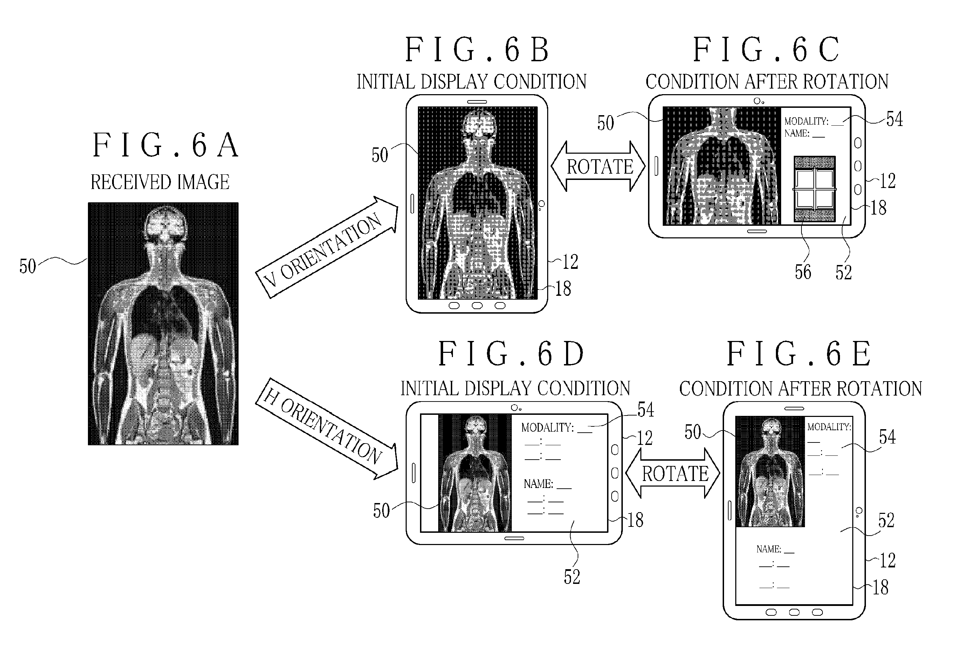

In case the image receiver 44 receives the medical image 50, the display control device 46 checks one of the horizontal and vertical orientations of the portable display unit 12 as illustrated in Fig. 4 according to the orientation information output by the orientation sensor 24. It is checked whether the display panel 18 extends horizontally or vertically. Then the display control device 46 resizes the medical image 50 by enlargement or reduction without changing the aspect ratio for an optimized size on the display panel 18 of the present orientation. The optimized size is the maximized size of the medical image 50 within the area of the display panel 18. See Figs. 6B and 6D.

After resizing the medical image 50, the display control device 46 drives the display panel 18 to display the medical image 50 in the resized form in a predetermined position, for example at an upper left corner. See Figs. 6B and 6D. It is checked whether a blank area 52 remains on the display panel 18 outside an area of the medical image 50. Assuming that the blank area 52 remains, then the display panel 18 is caused to display relevant information in the blank area 52. See Fig. 6D.

The relevant information is closely related to the medical image 50 displayed on the display panel 18. An example of the relevant information is auxiliary information 54 in the embodiment as illustrated in Fig. 6D. The auxiliary information 54 includes information of a method and date of examination and diagnosis, a name, age and sex of a patient, and the like, as attributes of the medical image 50. Assuming that the entirety of the medical image 50 cannot be displayed due to rotational shift of the portable display unit 12 or the like, a localizer 56 or localization information is displayed in the present embodiment as illustrated in Figs. 6C-6E. The localizer 56 is an indicia for informing a limited display area within the entire area of the medical image 50.

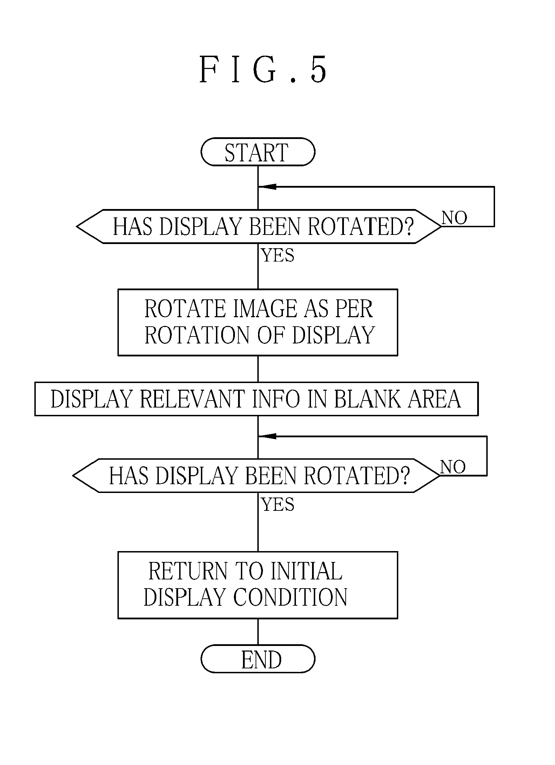

In Fig. 5, the display control device 46 monitors the orientation of the portable display unit 12 even after the medical image 50 of the resized form is displayed on the display panel 18. See Figs. 6B and 6D. In case rotational shift of the portable display unit 12 from one of the horizontal and vertical orientations to the remainder of those is detected, then the display control device 46 rotates the medical image relative to the display panel 18 so as to maintain an object orientation of the medical image. Thus, the object orientation of the medical image is recognized by a user in a rotationally unchanged state irrespective of the vertical or horizontal orientation of the portable display unit 12. In general, an orientation of a medical image is determined according to an orientation of expressing an imaged object. For example, an upper side of a medical image in which a head and chest of a patient are imaged is a head part in the medical image. Specifically, in case the portable display unit 12 is rotationally set in the vertical orientation, the longitudinal direction of the display panel 18 is the vertical direction. The display control device 46 determines an object orientation of the medical image so as to align the longitudinal direction of the display panel 18 with the vertical direction of the medical image. The rotation processing of the medical image is carried out for the determined obj ect orientation. In contrast, in case the portable display unit 12 is rotationally set in the horizontal orientation, the transverse direction of the display panel 18 is the vertical direction. The display control device 46 determines an object orientation of the medical image so as to align the transverse direction of the display panel 18 with the vertical direction of the medical image. The rotation processing of the medical image is carried out for the determined object orientation. See Figs. 6C and 6E.

The display control device 46 rotates the medical image 50 without changing the size. Assuming that the horizontal size of the medical image 50 after the rotation processing is larger than that of the display panel 18 after the rotational shift, or assuming that the vertical size of the medical image 50 after the rotation processing is larger than that of the display panel 18 after the rotational shift, then an end portion of the medical image 50 is removed to contain the medical image 50 within an area of the display panel 18 after the rotational shift. See Fig. 6C. Assuming that the horizontal size of the medical image 50 after the rotation processing is smaller than that of the display panel 18 after the rotational shift, or assuming that the vertical size of the medical image 50 after the rotation processing is smaller than that of the display panel 18 after the rotational shift, then the medical image 50 is slid to a predetermined corner of the display panel 18, for example an upper left corner. See Fig. 6E. After the rotation processing of the medical image 50, the display control device 46 causes the blank area 52 to display the relevant information after the blank area 52 is created by the rotation processing. See Figs. 6C and 6E.

Even after the portable display unit 12 is caused to make the first rotational shift (1/4 turn) described above, the display control device 46 monitors the orientation of the portable display unit 12. Assuming that the portable display unit 12 is caused to make a second rotational shift (1/4 turn) to return to the initial orientation, the display panel 18 is also returned to its initial display condition, as the medical image 50 is resized and displayed on the display panel 18. See Figs. 6B and 6D. The same operation is repeated. In case the portable display unit 12 is caused to make a (2n-1) th rotational shift of an odd number, the display panel 18 is set in the display condition after the first rotational shift of the portable display unit 12. See Figs. 6C and 6E. In case the portable display unit 12 is caused to make a (2n)th rotational shift of an even number, the display panel 18 is returned to its initial display condition. See Figs. 6B and 6D.

The operation of the above-described construction is described by referring to Figs. 6A-6E. Note that an aspect ratio of the medical image 50 distributed by the server apparatus 16 is equal to that of the display panel 18 of the portable display unit 12 of the vertical orientation according to Figs. 6A-6E.

In Fig. 6A, the orientation of the portable display unit 12 is detected upon distribution of the medical image 50 from the server apparatus 16. Assuming that the portable display unit 12 is in the vertical orientation, the medical image 50 is resized in a size equal to that of the display panel 18 of the vertically long shape (Fig. 6B), and displayed in the entire display area of the display panel 18. Then the portable display unit 12 is rotated to the horizontal orientation. As illustrated in Fig. 6C, the medical image 50 displayed on the display panel 18 of the vertically long shape is rotated without a change in the size, and slid to the left of the display panel 18. The relevant information is displayed in the blank area 52 to the right of the medical image 50. Furthermore, the portable display unit 12 is rotated to the vertical orientation, to return to the initial display condition of Fig. 6B.

An example of the auxiliary information 54 in Fig. 6C is as follows:
MODALITY: X-RAY
NAME: JOHN FUJI

Assuming that the portable display unit 12 is in the horizontal orientation upon transmitting the medical image 50, the medical image 50 is resized at a vertical size equal to that of the display panel 18 as illustrated in Fig. 6D, and displayed in a left portion of the display panel 18. Relevant information is displayed in the blank area 52 on a right side of the medical image 50. After this, the portable display unit 12 is rotated to the vertical orientation. In Fig. 6E, the medical image 50 on the display panel 18 of the horizontally long shape is rotated without changing the size, and slid to the upper left corner of the display panel 18. Relevant information is displayed in the blank area 52 extending on a right side and lower side from the medical image 50. The portable display unit 12 is further rotated to the horizontal orientation, and returns to the initial display condition of Fig. 6D.

An example of the auxiliary information 54 in each of Figs. 6D and 6E is as follows:
MODALITY: X-RAY
BODY PART: UPPER HALF
DATE: 18/2/2012
NAME: JOHN FUJI
AGE: 39
SEX: M

As described heretofore, the size of the medical image 50 is kept unchanged even in case the portable display unit 12 is rotated. This is effective in preventing failure in correctly recognizing the patient's progress of a lesion with a change in the size of the medical image 50. Also, the relevant information is displayed in the blank area 52 in the portable display unit 12. Thus, the entire display area can be utilized efficiently.

In the invention, the medical image 50 being displayed is rotated without changing the size according to the orientation of the portable display unit 12. Relevant information with the medical image 50 is displayed in the blank area 52 outside the medical image 50. Thus, details of the construction of examples can be changed and are not limited to the above embodiments.

For example, a command signal for enlargement or reduction of the medical image 50 can be input with the input interface 22, to enlarge or reduce a size of the medical image 50 being displayed. A shape and size of the blank area 52 is changed by the enlargement or reduction of the medical image 50. It is preferable to change a type, number and display condition of the relevant information according to the shape and size of the blank area 52.

In the above embodiment, the orientation of the portable display unit 12 is detected before initially displaying the medical image 50 on the display panel 18 after the distribution. Assuming that the portable display unit 12 is in the vertical orientation, the medical image 50 is resized in compliance with the display panel 18 of the vertically long shape. See Figs. 4 and 6B. Assuming that the portable display unit 12 is in the horizontal orientation, the medical image 50 is resized in compliance with the display panel 18 of the horizontally long shape. See Figs. 4 and 6D. The present invention is not limited to this embodiment.

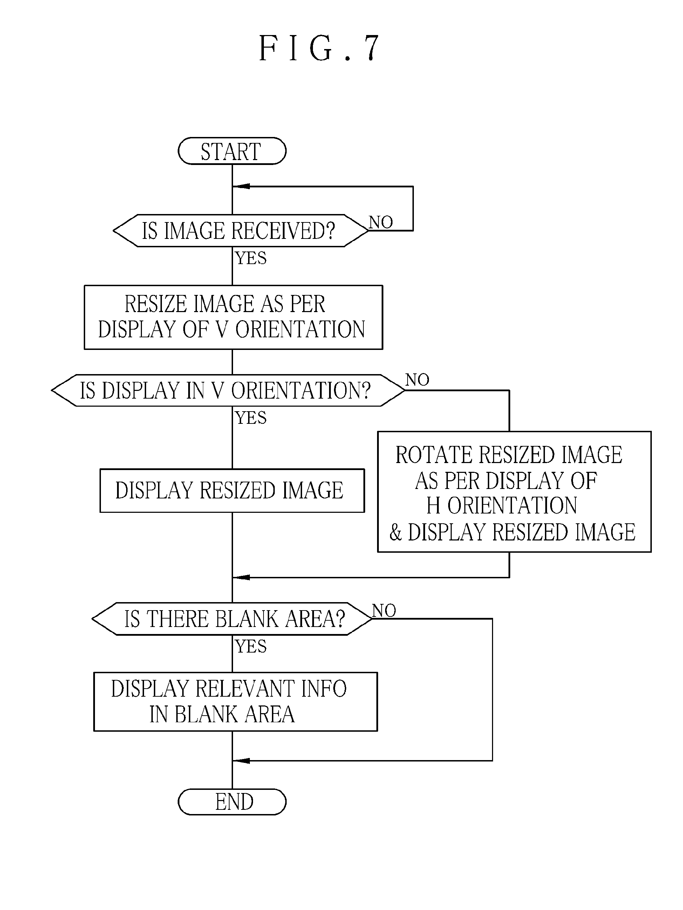

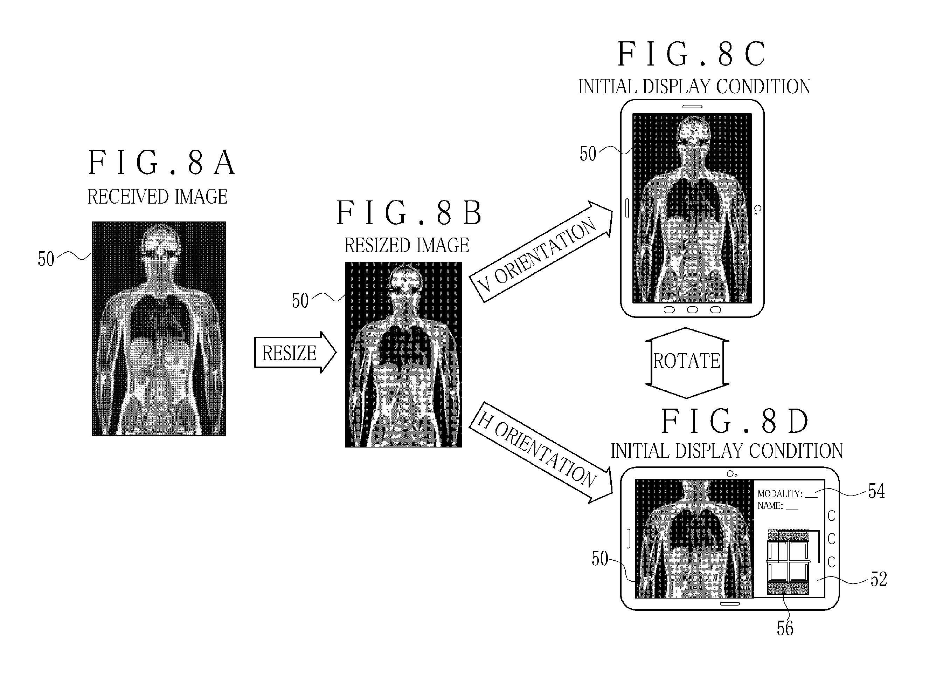

Also, it is possible as illustrated in Figs. 7 and 8A-8D to resize the medical image 50 in compliance with the display panel 18 of the vertically long shape irrespective of the orientation of the portable display unit 12 for the initially displaying step upon the distribution. This example of Figs. 8A-8D will be described in the condition in which an aspect ratio of the medical image 50 from the server apparatus is equal to an aspect ratio of the display panel 18 of the portable display unit 12 of the vertically long shape, in a similar manner to Figs. 6A-6E.

In case the medical image 50 of Fig. 8A is distributed, the display control device 46 of the portable display unit 12 resizes the medical image 50 in compliance with the display panel 18 of the vertically long shape as illustrated in Fig. 8B. Then the display control device 46 checks the orientation of the portable display unit 12, and assuming that the portable display unit 12 is in the vertical orientation, causes the display panel 18 to display the medical image 50 of the resized form as illustrated in Fig. 8C. Assuming that the portable display unit 12 is in the horizontal orientation, the display control device 46 cuts away at least one of upper and lower end portions of the medical image 50 after resizing in consideration of the vertical size of the display panel 18 of the horizontally long shape in Fig. 8D. The display control device 46 drives the display panel 18 to display the relevant information in the blank area 52. Also, in case the portable display unit 12 is rotated from the condition of Fig. 8C, the display control device 46 sets the portable display unit 12 in the condition of Fig. 8D. In case the portable display unit 12 is rotated from the condition of Fig. 8D, the display control device 46 sets the portable display unit 12 in the condition of Fig. 8C.

An example of the auxiliary information 54 in Fig. 8D is as follows:
MODALITY: X-RAY
NAME: JOHN FUJI

The display control according to the process in Figs. 7 and 8A-8D can enlarge a display size of the medical image 50 in comparison with the condition of the first embodiment in the case of the display panel 18 of the horizontally long shape in the initial display condition. See Figs. 6D and 8D. In case the portable display unit 12 is rotated from a condition of displaying the medical image 50 on the display panel 18 of the horizontally long shape, the display size of the medical image 50 can be enlarged. See Figs. 6E and 8C. Assuming that the display size of the medical image 50 is small, the medical image 50 is difficult to read. This problem of the difficulty is typically serious in the portable display unit 12 with a smaller form of the display panel 18 than a terminal apparatus of an installed type. Consequently, the medical image 50 can become easy to read by the display control according to Figs. 7 and 8A-8D.

In contrast, assuming that the display control is performed according to Figs. 7 and 8A-8D, the entirety of the medical image 50 cannot be displayed on the initial display condition for the display panel 18 of the horizontally long shape. See Fig. 8D. In general, however, most of the medical images 50 are in the vertically long shape. Many users or doctors with knowledge are likely to use the portable display unit 12 in the vertical orientation by considering the vertically long shape of the medical images 50. In short, possibility of the initial display condition in Fig. 8C is higher than that in Fig. 8D. It follows that advantage of the display control according to Figs. 7 and 8A-8D for enlarging the display size of the medical image 50 can be obtained more frequently than occurrence of the unwanted problem in insufficiently displaying the medical image 50 in the display panel 18 of the horizontally long shape.

In the above embodiment, the medical image 50 is resized before being displayed to cover the entirety of the medical image 50 in the initial display condition. In the example of Figs. 7 and 8A-8D, the medical image 50 is resized before being displayed to cover the entirety of the medical image 50 only assuming that the portable display unit 12 is in the vertical orientation. However, it is possible to cut away end portions of the medical image 50 protruding externally from the display panel 18 in the initial display condition without resizing. Furthermore, it is possible to predetermine a region of interest (ROI) for paying attention in viewing the medical image 50. The medical image 50 can be enlarged (resized) for easily viewing the region of interest, and the end portions of the medical image 50 outside the display panel 18 can be removed.

It is possible selectively to determine an area in the medical image 50 to be displayed and an area in the medical image 50 to be removed without display, on the condition of partially displaying the medical image 50. However, a difference between the centers of the medical image 50 before and after rotating the portable display unit 12 may hinder a user or operator from properly reading the medical image 50. Therefore, it is preferable to determine a selected one of areas in the entirety of the medical image 50 to be displayed so as to set a center of the medical image 50 after rotating the portable display unit 12 equal to a center of the medical image 50 before rotating the portable display unit 12.

In the course of display of the medical image 50 in the initial display condition, no problem occurs to reading the medical image 50 irrespective of positioning of the center of the medical image 50. It is possible to read the medical image 50 with great ease specially in case the center of the medical image 50 is the region of interest described above. According, it is preferable to determine a selected one of partial areas in the medical image 50 for display so as to set a region of interest at the center of the medical image 50 in the course of display of the medical image 50 in the initial display condition.

A region of interest can be determined for the medical image 50 by a suitable method. For example, a region of interest is determined at the time of imaging of the medical image 50. Information of the determined region of interest is stored as the auxiliary information 54 of the medical image 50. For this method, the display control device 46 of the portable display unit 12 refers to the auxiliary information 54 of the medical image 50 in response to transmission of the medical image 50, obtains the information of the region of interest, and performs the display control to locate the region of interest at the center of the medical image 50 displayed in the initial display condition.

In the above embodiment, the region of interest is determined at the time of imaging. However, a region of interest can be determined in the portable display unit 12. To this end, an image analysis device 60 or image analysis means (in Fig. 3) is incorporated in the portable display unit 12 for analyzing the medical image 50 being distributed and specifying an abnormal body part, which is set as a region of interest specified by the image analysis device 60. Furthermore, information of a diagnostic category (hospital department) can be utilized. In case the program for display control is started, a diagnostic category is selected, to determine a region of interest. For example, the display panel 18 is caused to display a list of a plurality of the diagnostic categories. The input interface 22 is operated to select one of the diagnostic categories. Assuming that the diagnostic category is cardiology or disease of the heart, the patient's chest is determined as a region of interest. Also, a touch panel structure can be utilized. A user can touch the medical image 50 on the touch panel structure to set a region of interest as a manual input.

Also, it is possible to keep the region of interest even upon displaying a new one of the medical images 50. For example, the medical image 50 with the region of interest at the chest is displayed first. A second one of the medical images 50 is newly displayed, and the region of interest at the chest is still determined for the second.

Furthermore, a region of interest may be changeable. For example, a doctor views a plurality of the medical images 50 in his or her predetermined sequence. The medical images 50 are viewed from a plurality of viewpoints. It is possible to redetermine the region of interest in the course of the sequence of the diagnosis. For example, he or she diagnoses a patient's brain in a sequence from the frontal lobe to the temporal lobe with a set of plural medical images. To this end, a first region of interest is determined at the frontal lobe in relation to a first medical image included in the medical images 50. A second region of interest is determined at the temporal lobe in relation to a second medical image immediately after the first medical image.

In the above embodiment, the size of the medical image 50 does not change even upon rotating the portable display unit 12. Another preferred embodiment of the display control of the medical image 50 is provided with a changeable size in the display panel 18. There are first and second control modes in the portable display unit 12 according to the program. In the first control mode, the medical image 50 is displayed at a fixed size without change even upon rotating the portable display unit 12. In the second control mode, the medical image 50 is enlarged or reduced in compliance with the display panel 18 assuming that the portable display unit 12 is rotated, and set at a maximum display size in the display panel 18 after the rotational shift. For changeover of the control modes, a user or operator can manipulate the input interface 22 for selecting a preferred one of the control modes.

In the above embodiments, the relevant information for display in the blank area 52 is the auxiliary information 54 or the localizer 56 of the medical image 50. However, the relevant information is not limited to the auxiliary information 54 or the localizer 56, but can be information relevant to the medical image 50. For example, extra images relevant to the medical image 50 can be the relevant information displayed in the blank area 52. Examples of the extra images are medical images included in an image group having the medical image 50 being displayed but different from this, a medical image of the same patient but obtained formerly in the examination of the same method, a medical image of the same patient obtained in a different examination, a medical image obtained in the examination of the same method from a patient different from the original patient. Also, such extra images can be preferably displayed as thumbnail images with a reduced size as relevant information. A set of the thumbnail images arranged together can include extra images and the medical image 50 of the original form with the reduced size.

Also, a link tag can be used in place of the thumbnail form of the medical image 50 as relevant information. The link tag is displayed in the blank area 52 for accessing a location of storage of the medical image 50 (link destination). The medical image 50 associated with the link tag can be newly displayed in place of a presently displayed one of the medical images 50 by selecting the link tag according to user preference. Furthermore, the medical image 50 itself can constitute a link tag while displayed. The medical image 50 as relevant information is directly selected or designated to display a second one of the medical images 50 by changeover.

Also, association with the link tag is not limited to the medical image 50. For example, address data of reference information relevant to the medical image 50 being displayed can be associated with a link tag. The reference information can be displayed on the display panel 18 in response to selecting the link tag. For example, the reference information can be an Internet address of a manual for describing a list of symptoms of the chest for an X-ray image of the chest and medically distinct features of the symptoms in the case of the chest as an object of the displayed X-ray image. Accordingly, diagnosis with the medical image 50 can be more efficient.

Note that the medical image 50 is disposed in the left side in Figs. 6C and 8D, but can be disposed in a right side or at the center on the display panel 18. The blank area 52 can be formed according to the arrangement of the medical image 50. The medical image 50 is formed at the upper left corner in Fig. 6E, but can be formed at one of the other corners or in an intermediate position between the corners.

In Fig. 6A, the medical image 50 is in the vertically long shape. The portable display unit 12 is in the vertical orientation in Fig. 6B, and becomes in the horizontal orientation in Fig. 6C after being rotated. However, it is likely initially that the medical image 50 is in the horizontally long shape and that the portable display unit 12 is in the horizontal orientation. The portable display unit 12 becomes in the vertical orientation in case the same is rotated. The medical image 50 is disposed in an upper side on the display panel 18. Alternatively, the medical image 50 may be disposed in a lower side on the display panel 18, or at the center on the display panel 18. The blank area 52 can be formed according to the arrangement of the medical image 50.

Note that the medical image in the present invention can be any images, and can be formed by imaging systems of various modalities. For example, the medical image can be an X-ray image, and an image formed by the computed tomography (CT), magnetic resonance imaging (MRI), or ultrasound echo imaging. Also, the medical image can be electrocardiogram information and information of a graph of other types. Moreover, a field of the diagnosis of the invention may be a field other than the medical field, for example, industrial field and social research field. A data format of the medical image can be JPEG, PDF, TIF and the like readable electronically.

In the above embodiments, the display panel 18 can be any of various light valve devices, for example, a liquid crystal display panel, cathode ray tube (CRT), electroluminescence device (EL) and the like. The portable display unit 12 as a mobile terminal apparatus is a computer of a tablet type or notebook type. However, a portable display unit as a mobile terminal apparatus of the invention may be a laptop computer, cellular phone, smart phone, portable digital assistant (PDA), mobile Internet device (MID), digital broadcast receiver, and the like.

The network connection can be made wirelessly or in a wired manner. Also, the portable display unit 12 and the server apparatus 16 for images can be connected to one another without the network, for example, by use of a USB cable in a suitably wired manner.

For the purpose of transmitting a medical image, an improper access should be inhibited. To this end, a security technique well-known in the field can be utilized for authorizing access of the portable display unit 12 to the server apparatus 16.

Furthermore, the feature of the present invention is applicable not only to the portable display unit but also to a display method, a computer-executable program for display, and a storage medium for storing the computer-executable program.

Although the present invention has been fully described by way of the preferred embodiments thereof with reference to the accompanying drawings, various changes and modifications will be apparent to those having skill in this field. Therefore, unless otherwise these changes and modifications depart from the scope of the present invention, they should be construed as included therein.

## Claims

1. A portable display unit comprising:
a display panel (18), having a predetermined aspect ratio, for displaying a medical image (50);
an orientation sensor (24) for detecting a selected one of a horizontal orientation and a vertical orientation in which said display panel is positioned, wherein a longitudinal direction of said display panel is vertical in case of said vertical orientation, and is horizontal in case of said horizontal orientation;
a display control means (46) for processing said medical image in rotation processing relative to said display panel by maintaining a size and an obj ect orientation of said medical image assuming that said display panel is rotationally shifted from one of said horizontal and vertical orientations to a remaining one thereof, wherein assuming that a blank area (52) is created outside a display area of said medical image on said display panel after said rotation processing, said display control means displays relevant information (54, 56) relevant to said medical image in said blank area.

2. A display unit as defined in claim 1, wherein assuming that an end portion of said medical image protrudes from said display panel after said rotation processing, said display control means causes said display panel to display said medical image after removing said end portion.

3. A display unit as defined in claim 1 or 2, wherein said display control means resizes said medical image to be displayed on said display panel.

4. A display unit as defined in claim 3, wherein said display control means processes said medical image being resized in said rotation processing.

5. A display unit as defined in any one of claims 1 to 4, wherein said display control means resizes said medical image to display said medical image entirely in said vertical orientation;
assuming that said display panel is in said vertical orientation, said medical image after being resized is displayed;
assuming that said display panel is in said horizontal orientation, said display control means creates a partial image by removing at least one of upper and lower end portions of said medical image after being resized, and causes said display panel to display said partial image after said rotation processing.

6. A display unit as defined in any one of claims 1 to 5, wherein said relevant information is auxiliary information attached to said medical image being displayed.

7. A display unit as defined in any one of claims 1 to 6, wherein said relevant information is a localizer, displayed in case said display area of said medical image is partially displayed on the display panel, for indicating a position and size of said display area.

8. A display unit as defined in any one of claims 1 to 7, wherein said relevant information is a thumbnail image obtained by reducing a size of one or more other medical images related to said medical image being displayed.

9. A display unit as defined in any one of claims 1 to 8, wherein said relevant information is a link tag for access to a link destination for storing information related to said medical image being displayed.

10. A display unit as defined in any one of claims 1 to 9, wherein a region of interest is determined in said medical image, and said display control means performs control for said medical image to dispose said region of interest at a center of said display area of said medical image.

11. A display unit as defined in claim 10, further comprising an input interface means, externally operable, for determining said region of interest.

12. A display unit as defined in claim 10 or 11, further comprising an image analysis means for analyzing said medical image, wherein said region of interest is determined according to analysis in said image analysis means.

13. A display unit as defined in any one of claims 1 to 12, further comprising an input interface means for selectively setting first and second control modes for operating said display control means;
wherein in said first control mode, said size of said medical image is fixed in said rotation processing, and in said second control mode, said size of said medical image is adjustable in said rotation processing.

14. A display unit as defined in claim 13, wherein in said second control mode, said display control means enlarges or reduces said medical image being displayed according to said aspect ratio of said display panel after said rotation processing, and displays said medical image in a maximized size in said display panel after said rotation processing.

15. A display unit as defined in any one of claims 1 to 14, further comprising a communication interface for connection to a server apparatus by network connection and receiving transmission of said medical image from said server apparatus.

16. A display unit as defined in any one of claims 1 to 15, wherein said orientation sensor is a gyro sensor or magnetic sensor.
